# EUROPEAN PATENT SPECIFICATION

(11) **EP 0 762 123 B1**
(45) Date of publication and mention of the grant of the patent: **05.12.2001**
(21) Application number: 96114299.9
(22) Date of filing: 06.09.1996
(51) Int. Cl.: G01N 33/558, G01N 33/571

(54) **Immunoassay device and immunoassay method using the same**
Immunoassay Vorrichtung und Testverfahren zu seiner Verwendung
Dispositif d'analyse immunologique et méthode d'analyse l'utilisant

(30) Priority: 08.09.1995 JP 25675695; 08.09.1995 JP 25675795; 20.12.1995 JP 34852895
(43) Date of publication of application: 12.03.1997
(73) Proprietor: FUJIREBIO INC., Tokyo 103-0007 (JP)
(72) Inventor: Saruta, Hiroko, Shinjuku-ku, Tokyo (JP); Hasegawa, Akira, Shinjuku-ku, Tokyo (JP); Ashihara, Yoshihiro, Shinjuku-ku, Tokyo (JP); Fujiwara, Yuko, Shinjuku-ku, Tokyo (JP); Isomura, Mitsuo, Shinjuku-ku, Tokyo (JP)
(74) Representative: Hansen, Bernd, Dr. Dipl.-Chem.

(56) References cited:
- EP-A- 0 299 428
- EP-A- 0 381 173
- EP-A- 0 512 390
- EP-A- 0 585 914
- EP-A- 0 670 494
- WO-A-91/12528

## Description

### BACKGROUND OF THE INVENTION

This invention relates to an immunoassay device to be used for detecting an antigen or antibody in a specimen, and an immunoassay method using the same.

This invention also relates to an immunoassay method which comprises adding a specimen to an immunoassay device in which a labelling reagent zone comprising a labelled genetic recombinant syphilis treponema (*Treponema pallidum*, hereinafter referred to as "TP") antigen and a detection zone in which a TP antigen is immobilized to a matrix which can transfer a solution by capillarity are provided on the matrix; and detecting an anti-syphilis treponema antibody (hereinafter referred to as "the anti-TP antibody") in the specimen which is bound to the detection zone, and an immunoassay device to be used for said assay.

As a device which detects an antigen or antibody in a specimen simply and easily, an immunoassay device has been used. A conventional immunoassay device is schematically shown in Fig. 10 (see unexamined Japanese Patent application with Publication No. 47894/1978). The conventional immunoassay device has a membrane portion 20 comprising a material such as cellulose, and a developing solution-supplying portion 22 and an absorption portion 24 each comprising a water-absorbable material, provided at both ends of the membrane portion 20, respectively. In the membrane portion 20, a labelled substance dotting portion 26 to which a labelled substance labelled with a radioisotope is dotted is provided. Further, a detection line 28 is provided between the labelled substance dotting portion 26 and the absorption portion 24. At the detection line 28, an antibody or antigen which reacts with an antigen or antibody to be detected is immobilized to the membrane. Further, between the labelled substance dotting portion 26 and the developing solution-supplying portion 22, a specimen dotting portion 29 is provided.

When the device is used, a specimen solution is added to the specimen dotting portion 29, and a developing solution is added to the developing solution-supplying portion 22. The developing solution is supplied to the device via the developing solution-supplying portion 22 and reaches to the specimen dotting portion 29. The specimen dotted to the specimen dotting portion 29 is conveyed by the developing solution flowing from the developing solution-supplying portion 22, reaches to the labelled substance dotting portion 26 and reacts with the labelled substance. The reaction mixture is further carried by the developing solution to the detection line 28, where an antigen or antibody in the specimen is trapped by an antibody or antigen immobilized to the detection line 28 and held there. In this case, the antigen or antibody in the specimen is bound to the labelled substance so that the labelled substance is also held at the detection line 28. Therefore, when the antigen or antibody to be detected is contained in the specimen solution, the signal of the radioisotope is observed on the detection line 28. The developing solution and other components which are not trapped are absorbed into the absorption portion 24. On the other hand, when the antigen or antibody to be detected is not contained in the specimen solution, the labelled substance is not trapped at the detection line 28 but is absorbed into the absorption portion 24, so that no signal is observed on the detection line 28. Thus, whether or not the antigen or antibody to be detected is contained in the specimen can be determined by investigating whether or not a signal of the radioisotope is detected at the detection line 28.

However, in the conventional immunoassay device as described above, there are problems related to the fact that since the specimen dotted to the specimen dotting portion 29 is diluted with the developing solution during measurement, a lowering of detection sensitivity is caused, and since the labelled substance is dissolved in the developing solution during development and reacts with the specimen, when an object to be measured has low concentration, in order to carry out an accurate test, a long reaction time is necessary.

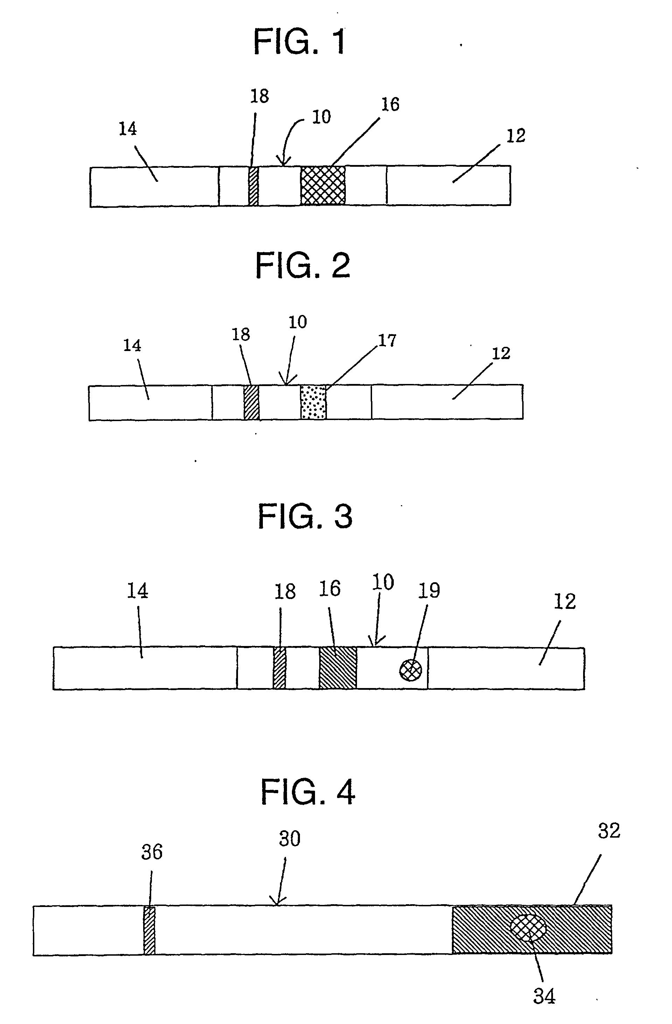

An immunoassay device using a color latex has been also known. In this device, color latex particles are used as a labelled substance to which an antibody or antigen which reacts with an antigen or antibody to be detected is bound. This device is schematically shown in Fig. 4. A labelled substance dotting portion 32 is provided at one end of a membrane portion 30, and the above labelled substance is contained in the labelled substance dotting portion 32. On the other hand, an antibody or antigen which reacts with an antigen or antibody to be detected is immobilized to a detection line 36. A specimen solution is added to a sample dotting portion 34 in the labelled substance dotting portion 32. The specimen solution and the labelled substance dissolved by the specimen solution may react with each other whilst they migrate, and when an antigen or antibody to be detected is contained in the specimen solution, the labelled substance is trapped at the detection line 36 as in the above case. The labelled substance contains a color latex so that the detection line 36 is colored. On the other hand, when the antigen or antibody to be detected is not contained in the specimen solution, the labelled substance is not trapped at the detection line 36 so that the detection line 36 is not colored. Thus, whether or not the antigen or antibody to be detected is contained in the specimen solution can be determined by assessing whether or not the detection line 36 is colored.

However, the conventional immunoassay devices suffer from the problem that an initially negative result may be reversed to a positive result over the course of time. Therefore, when plural specimens are analyzed under the same conditions, it is necessary to carry out the assessment at a specified time or to carry out the assessment after the reaction is terminated by adding a reaction-terminating solution after the lapse of a specified period of time. However, when a large number of specimens is tested simultaneously as in clinical tests, it is difficult to carry out the assessment of all specimens at the same time or to add a reactronterminating solution after a lapse of the same period of time. Further, when a reaction-terminating solution is added, the number of steps increases, making the operation troublesome.

It is important to analyze living body components or drugs contained in blood, urine and the like for the diagnosis of diseases and for the judgment of therapeutic progress. As a method for analyzing these living body components, drugs and the like from a specimen simply and easily by utilizing an antigen-antibody reaction, there has been developed a method of using a strip assay device comprising a strip-shaped filter paper impregnated with a reaction reagent. This assay method is a method in which a specimen is added to a specimen dotting zone provided on the filter paper of the device, a solution is developed and the analysis is carried out by assessing the degree of coloring shown at the detection zone provided on the filter paper. In the above strip assay device, a filter paper containing a necessary reagent (an enzyme-labelled antibody, a substrate, a coloring reagent or the like)-depending on the system of a reaction is used, and the analysis is carried out on the basis of the coloring thereof thus providing a simple and easy method without the need of a special judgment device. Also, there has been known an assay method of using a color latex or particles of metal colloid or the like as a labelled substance. In this assay method, analysis is carried out by using a reagent in which an antibody or antigen which reacts with an antigen or antibody to be detected is bound to particles and detecting the amount of particles bound to a detection zone.

In the prior art, as a method for detecting the anti-TP antibody, there has been known a method of using a TPHA reagent in which a TP cell component is bound to hemocytes, a method of using an indirect agglutination immunoassay reagent produced by binding a TP antigen to artificial particles and a method of using an immunoassay reagent comprising a TP antigen-binding solid phase and a labelled anti-globulin antibody. In all of these methods, 2 hours or more are required from the beginning of the measurement to obtaining a result, and a detection device is necessary so that the methods cannot be used for an emergency test or for the measurement at a bedside within a short period of time.

In the methods for measuring the anti-TP antibody using conventional strip assay devices, large amounts of globulin existing in a specimen may react with the labelled antibody, thus impeding the measurement. Therefore, there has been found a method of using a device in which a washing reservoir is added to an assay device (see unexamined Japanese Patent application with Publication No. 126832/1993) and a method of using a device in which a zone for preventing signals generated by a labelled substance is provided in a developing solution (WO 88/08536 A; JP 503439/1989 A; JP 2,599,192 B).

These methods are complicated to operate, and it is difficult to maintain constant operating conditions at all times. Further, due to influence of globulin or the like contained in a specimen, the methods do not allow to carry out measurement with a good sensitivity. For the measurement of the anti-TP antibody in a specimen it has been attempted to solve these problems by detecting said antibody using a TP antigen and a labelled TP antigen bound to a solid phase according to the sandwich method. However, the prior art teaches no easy way of introducing a labelled substance into a mixture containing a TP antigen obtained by culturing in a living body. Moreover, a labelled antigen reagent which was substituted by a constant labelled substance at all times could not be obtained continuously. As described above, a method of using a labelled TP antigen has not been known.

In the conventional methods for measurement of the anti-TP antibody, there are problems associated with the complicated operation and because an analysis with a high precision cannot be carried out within a short period of time.

### SUMMARY OF THE INVENTION

A first object of the present invention is to provide an immunoassay device in which detection sensitivity is high and an accurate test can be carried out within a shorter period of time as compared with the conventional immunoassay devices, and to provide an immunoassay method using the same.

A second object of the present invention is to provide an immunoassay device in which an initially negative result is not reversed to a positive result with a lapse of time, and to provide an immunoassay method using the same.

A third object of the present invention is to provide an assay method by which a result can be obtained within a short period of time which is simple and easy to operate as compared with conventional methods, and to provide a device for practicing said assay.

The present inventors have studied intensively and consequently found that the low sensitivity of conventional assay devices is caused by problems relating to the facts that a specimen dotted to a specimen dotting portion is diluted before it reacts with a labelled substance, that the labelled substance dotted to a labelled substance dotting portion cannot react to a sufficient extent with the specimen dotted to a specimen dotting portion, and that the amount of a specimen solution dotted to a specimen dotting portion is small. It has been found that these problems can be solved by incorporating a labelled substance on a membrane, to accomplish the present invention.

Also, the present inventors have studied intensively and consequently found that the above second object can be achieved not by incorporating a substrate of an enzyme into a developing solution as in the prior art, but by incorporating a substrate of an enzyme into the membrane in a dry state, to accomplish the present invention.

That is, the present invention provides an immunoassay device as described in Claim 1.

The present inventors have studied intensively and consequently found an immunoassay method as disclosed in Claim 8. In a preferred embodiment, the specimen is added to an immunoassay device for an anti-syphilis treponema antibody, in which a labelling reagent zone comprising a labelled genetic recombinant TP antigen and a detection zone in which a TP antigen is immobilized to a matrix which can transfer a solution by capillarity are provided on the matrix. This allows the detection of anti-syphilis treponema antibodies in the specimen bound to the detection zone. A novel immunoassay device for the anti-TP antibody is also disclosed, in which a labelling reagent zone comprising a labelled genetic recombinant TP antigen is provided on a matrix which can transfer a solution by capillarity, to accomplish the present invention. More specifically, they have found an immunoassay method which comprises the use of an immunoassay device for the anti-TP antibody, in which a developing solution zone containing a substrate, a labelling reagent zone comprising a genetic recombinant TP antigen labelled with an enzyme, a detection zone in which a TP antigen is immobilized to a matrix which can transfer a solution by capillarity, a specimen dotting zone and a developing solution-absorbing zone are provided on the matrix; dotting a specimen solution to the specimen dotting zone; supplying a developing solution to the developing solution zone; and detecting the enzyme immobilized to the detection zone in an amount depending on the anti-TP antibody in the specimen solution. Another embodiment of the present invention relates to an immunoassay method which comprises using an immunoassay device for the anti-TP antibody, in which a specimen developing solution zone for adding a developing solution containing a specimen and a genetic recombinant TP antigen labelled with a radioisotope, a latex, metal colloid particles, fluorescent particles or colored particles, a detection zone in which a TP antigen is immobilized to a matrix which can transfer a solution by capillarity and a developing solution-absorbing zone are provided on the matrix; adding a specimen solution to the specimen dotting zone; supplying a developing solution to the developing solution zone; and detecting the labelled substance immobilized to the detection zone in an amount depending on the anti-TP antibody in the specimen solution.

### BRIEF DESCRIPTION OF THE DRAWINGS

Fig. 1 is a view showing schematically one example of an embodiment of the immunoassay device using a labelled substance pad of the present invention.

Fig. 2 is a view showing schematically the immunoassay device of the present invention in which a labelled substance is dotted to a membrane portion.

Fig. 3 is a view showing schematically one example of a preferred embodiment of the immunoassay device of the present invention.

Fig. 4 is a view showing schematically a conventional immunoassay device using a color latex.

Fig. 5 is a plane view showing one example of an embodiment of the immunoassay device of the present invention.

Fig. 6 is a sectional view of the immunoassay device of the present invention having in addition a labelling reagent zone comprising a pad containing a labelled genetic recombinant TP antigen.

Fig. 7 is a sectional view of the immunoassay device of the present invention wherein a substrate zone 7 is provided on a matrix 1.

Fig. 8 is a sectional view of the immunoassay device of the present invention wherein two detection zones 5 and 8 are provided on a matrix.

Fig. 9 is a plane view of the immunoassay device of the present invention wherein the measurement is carried out by supplying a mixed solution 9 of a specimen and a genetic recombinant TP antigen labelled with a radioisotope, a latex, metal colloid particles, fluorescent particles or colored particles to a developing solution zone 2.

Fig. 10 is a view showing schematically a conventional immunoassay device.

### DESCRIPTION OF THE PREFERRED EMBODIMENTS

In the following, the present invention is explained in detail.

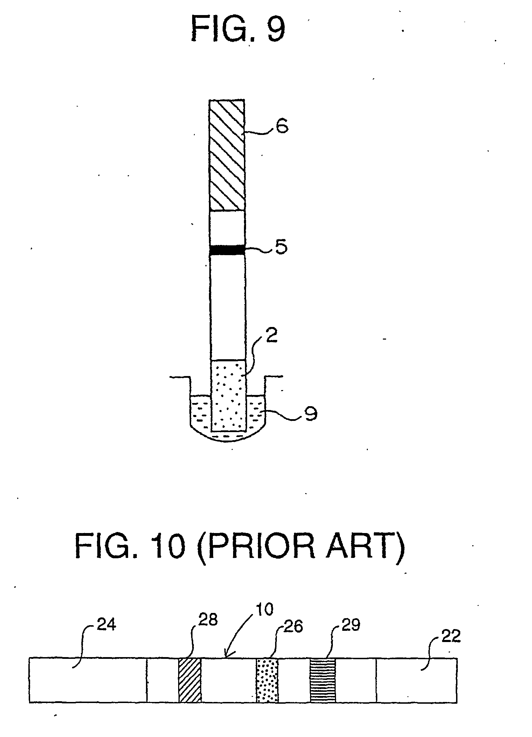

A preferred first embodiment of the immunoassay device of the present invention is schematically shown in Fig. 1 and Fig. 2. Its basic constitution is the same as that of the conventional assay device shown in Fig. 10. That is, the assay device of the present invention has a membrane portion 10. As in the prior art, the membrane portion 10 generally comprises a material such as a membrane formed from nitrocellulose, cellulose or glass fiber and generally has a rectangular shape. At both ends of the membrane portion 10, a developing solution-supplying portion 12 and an absorption portion 14 are provided, respectively. In these portions, the membrane can be elongated, or a water-absorbing material such as a sponge, a water-absorbing nonwoven fabric and a filter paper can be added, as described in the prior art. The developing solution-supplying portion 12 and the absorption portion 14 are generally formed to be thicker than the membrane portion 10 so that they can absorb a large amount of a liquid, which, however, is not indispensable. A detection portion 18 is provided between the labelled substance dotting portion 16 or 17 (described below) provided on the membrane portion 10 and the absorption portion 14. As in the prior art, the detection portion 18 contains an immobilized antibody or antigen which reacts with the antigen or antibody to be detected. As in the prior art, the detection portion 18 is preferably formed to have a linear shape (line(s)), but the shape is not necessarily linear and may be different, for instance circular. When the detection portion 18 has a linear shape, a control line which is colored such as red, blue, etc., for confirming development of a developing solution reaching said portion may additionally be provided at the downstream side of the line for detection. Also, as in the prior art, the membrane portion 10 is blocked with BSA (bovine serum albumin) or the like in order to prevent nonspecific adsorption of protein.

In the assay device of the first embodiment shown in Fig. 2 of the present invention, the labelled substance dotting portion 17 is provided on the membrane portion 10. In the labelled substance dotting portion 17, a labelled substance an antibody or antigen which is capable binding the anti- gen or antibody to be detected is labelled with an enzyme is contained in a dry state wherein an antibody or antigen which is capable of binding to the antigen or antibody to be detected is labelled with an enzyme. In the assay device shown in Fig. 1 of the present invention, a labelled substance pad 16 formed from a water-absorbable material, containing the labelled substance may be provided on the membrane portion 10 as the labelled substance dotting portion. As in the prior art, as a labelling enzyme, there may be used alkaline phosphatase, peroxidase, β-galactosidase, β-glucosidase and the like which have been used frequently in immunoassays. Further, the labelled substance can be prepared by binding the enzyme to the antigen or antibody by covalent bonding or non-covalent bonding.

The material of the labelled substance pad 16 is not particularly limited so long as it absorbs water, and there may be mentioned a sponge, a water-absorbing nonwoven fabric and a filter paper. The size of the labelled substance pad 16 is not particularly limited, but it has generally a length and a width of about 3 to 10 mm and a thickness of about 0.5 mm to 4 mm. The amount of the labelled substance to be contained in the labelled substance pad 16 is not particularly limited so long as it is higher than the amount necessary when the labelled substance is directly dotted to a conventional membrane and dried, and it is generally about 0.01 to 5 µg in terms of dry weight although it may vary depending on the respective tests.

When the device is used, a specimen solution is added to the labelled substance dotting portion 17 or the labelled substance pad 16, and a developing solution is added to the developing solution-supplying portion 12. The developing solution contains a substrate of the labelling enzyme of the labelled substance, which, for example, becomes colored upon reaction with the enzyme. The labelled substance contained in the labelled substance dotting portion 17 or the labelled substance pad 16 reacts with an antigen or antibody in the specimen solution, is conveyed by the developing solution flowing from the developing solution pad portion 12 and reacts with the substrate in the developing solution to produce a pigment. When they are transported by the developing solution to the detection portion 18, the antigen or antibody in the specimen is trapped by an antibody or antigen immobilized to the detection portion 18 and held there. In this case, the antigen or antibody in the specimen is bound to the labelled substance so that the labelled substance is also held at the detection line(s) of the detection portion 18 and reacts with the substrate to effect pigmentation. Between the developing solution-supplying portion 12 and the detection portion 18, the substrate moves in the developing solution while it reacts with the labelled substance, and at the detection portion 18, a sufficient amount for carrying out immunoassay of the substrate is contained in the developing solution. Therefore, when the antigen or antibody to be detected is contained in the specimen solution, coloring is observed on the detection portion 18. The developing solution and the pigment and other components which are not trapped are absorbed into the absorption portion 14. On the other hand, when the antigen or antibody to be detected is not contained in the specimen solution, the labelled substance is not trapped at the detection portion 18 and therefore is absorbed as such into the absorption portion 14, whereby coloring is not observed on the detection portion 18.

Thus, whether or not the antigen or antibody to be detected is contained in the specimen can be found by determining whether or not the detection portion 18 is colored.

As the substrate contained in the developing solution, there may be mentioned various coloring substrates corresponding to the above enzyme, for example, 3-indolyl derivatives such as 5-bromo-4-chloro-3-indolyl phosphate, 5-bromo-6-chloro-3-indolyl phosphate, 5-bromo-4-chloro-3-indolyl-β-D-galactopyranoside, 5-bromo-6-chloro-3-indolyl-β-D-galactopyranoside, 5-bromo-4-chloro-3-indolyl-β-D-glucopyranoside and 5-bromo-6-chloro-3-indolyl-β-D-galactopyranoside.

In the above explanation, as the substance to be labelled with the enzyme and the substance to be immobilized to the detection portion 18, an antibody or antigen is used. However, "an antibody or antigen" mentioned in the present specification includes an antibody fragment (e.g., a Fab fragment and a F(ab')₂ fragment), hapten and the like so long as they are susceptible to an antigen-antibody reaction.

In the assay device of the present invention, the specimen is sufficiently reacted with the labelled substance without dilution, and an immune complex of the labelled substance and the antigen or antibody in the specimen is formed. Further, in the assay device of the present invention, when the above labelled substance pad is used, the amount of the labelled substance to be contained can be increased to twice or more of the conventionally used amount. Moreover, the labelled substance is transported more smoothly by the developing solution as compared with the case where a labelled substance is directly dotted to a membrane and dried. Further, the amount of the specimen solution to be used can be increased to about five times of the amount conventionally used. That is, in an assay device using a labelled substance pad, even when a large amount of a specimen solution is added to a labelled substance dotting portion, a membrane cannot absorb a large amount of the specimen solution so that the specimen solution is protuberant on the membrane and flowed out to the outside of the device. The specimen solution cannot be utilized effectively, so that it is meaningless to use a large amount of the specimen. Therefore, the amount of the specimen solution to be added is generally about 5 µl, and not a conventional dropping pipette, but a special dispenser is used. On the contrary, in the assay device of the present invention which uses a labelled substance pad, the specimen solution is added to the water-absorbable labelled substance pad so that a larger amount of the specimen solution can be absorbed as compared with a conventional case where a specimen solution is added to the specimen dotting portion 29 which is a membrane. Due to the above facts, in the assay device of the present invention, detection sensitivity is high, and an accurate test can be carried out within a shorter period of time as compared with the conventional device. Further, about 25 µl of the specimen solution can be added so that a conventional dropping pipette can be used, which results in a simple and easy operation.

A preferred second embodiment of the immunoassay device of the present invention is schematically shown in Fig. 3. Its basic constitution is the same as that of the above conventional assay device shown in Fig. 2.

In the labelled substance-containing portion 16, a labelled substance is contained in a dry state wherein an antibody or antigen which reacts with an antigen or antibody to be detected is labelled with an enzyme. As in the prior art as a labelling enzyme, there may be used alkaline phosphatase, peroxidase, β-galactosidase and the like which have been used frequently in immunoassays. The labelled substance may be directly dotted to the membrane, or a labelled substance pad comprising a water-absorbing material such as a sponge, a water-absorbing nonwoven fabric and a filter paper may be provided on the membrane, and the labelled substance may be incorporated into the labelled substance pad. In this case, the labelled substance can be contained in a larger amount, the labelled substance is easily transported by a developing solution, and a large amount of the specimen solution can be used, thus allowing the detection sensitivity to be increased.

In the immunoassay device of the present invention, a substrate-containing portion 19 containing a substrate of the labelling enzyme in a dry state is provided on the membrane portion 10, which is different from the conventional devices. The amount of the substrate to be contained in the substrate-containing portion 19 is not particularly limited, but it is generally about 20 to 200 µg although it may vary depending on the respective tests. As in the prior art, the substrate is a substrate of a labelling enzyme, which becomes colored upon reaction with the enzyme.

When the device is used, a specimen solution is added to the labelled substance-containing portion 16, and a developing solution is added to the developing solution pad portion 12. Contrary to the teaching of the prior art the substrate of the labelling enzyme of the labelled substance is not contained in the developing solution. The developing solution added to the developing solution pad portion 12. It flows to the substrate-containing portion 19 where it dissolves the substrate and subsequently reaches the labelled substance-containing portion 16. The subsequent procedure is the same as that shown in Fig. 1.

In the conventional devices, the substrate is contained in the developing solution, and a large amount of the developing solution is used so that the coloring reaction continues for an indefinite period of time. On the contrary, in the assay device of the present invention, the coloring reaction does not proceed any more after the entire amount of the substrate contained in the substrate-containing portion 19 is further transported away by the developing solution, so that no more substrate is available. Therefore, a result which was determined to be negative at the time of assessment is not reversed to positive over the course of time. Further, in the assay device of the present invention, the substrate is maintained in a dry state so that its storage stability is high as compared to the case where the substrate is kept dissolved in the developing solution, as taught in the prior art.

Next, as the third embodiment of the present invention, the immunoassay method of the present invention is described in detail by referring to an immunoassay device. In the immunoassay device, the matrix comprises a water-absorbing material which can transfer a solution by capillarity. A preferred material includes, for example, cellulose or a derivative thereof such as cellulose and nitrocellulose, a filter paper formed from a glass fiber or the like and a porous film. The size of the matrix is not limited, but, for example, a strip-shaped matrix having a width of about 3 mm to 10 mm and a length of about 30 mm to 100 mm is preferred since its handling is easy. The thickness of the matrix is preferably 100 µm to 1 mm. A part of the matrix may be blocked with bovine serum albumin (BSA), casein, sucrose or the like in order to prevent the adsorption of proteins by a nonspecific reaction.

### (Labelling reagent zone)

The labelling reagent zone is a zone containing a labelled genetic recombinant TP antigen provided on the matrix. This zone can be provided at a position which is upstream to the detection zone with regard to the transferring direction of the developing solution from the developing solution zone. This zone can be provided by dotting a labelled genetic recombinant TP antigen to the matrix or by providing a water-absorbable pad containing a labelled genetic recombinant TP antigen on the matrix.

As the TP antigen, surface antigens having various molecular weights exist on the cell surface of TP, and as a main example, antigens having molecular weights of 47 kDa (TP47), 42 kDa (TP42), 17 kDa (TP17) and 15 kDa (TP15) are known. Genes coding these antigens have already been cloned, and the antigens have been produced by genetic engineering (WO-A-500403/1990, INFECTION AND IMMUNITY, Vol. 57, No. 17, pp.
3708 to 3714, 1989 and Molecular Microbiology, 4(8), pp. 1371 to 1379, 1990). The genetic recombinant TP antigen of the present invention can be prepared by culturing *Escherichia coli* which has been transformed by integrating the above antigen having the respective molecular weight into a vector according to the method of Norgard et al. (INFECTION AND IMMUNITY, Vol. 61, pp. 1202 to 1210, 1993) and carrying out the purification from a culture solution by combining known methods. In the present invention a genetic recombinant TP antigen may be used which is a derivative of the above antigen protein having the respective molecular weight. For example, a TP antigen in which glutathione-S-transferase (GST) is fused to the N end of protein, produced by genetic engineering (see unexamined Japanese Patent application with Publication No.287017/1995) may be used.

The genetic recombinant TP antigen is bound to a labelled substance. As the labelled substance, there may be mentioned, for example, an enzyme, a radioisotope, a latex, metal colloid particles, fluorescent particles and colored particles. As the enzyme, there may be mentioned various kinds of enzymes used in enzyme immunoassay (EIA), for example, alkaline phosphatase, peroxidase and β-D-galactosidase. As the radioisotope, there may be mentioned, for example, an isotope such as iodine, tritium and carbon. As the latex, there may be mentioned, for example, particles of a high molecular weight compound such as a polystyrene latex. The metal colloid particles are, for example, particles comprising various kinds of metal colloids, and there may be mentioned colloid of a metal such as selenium, platinum and gold. The particle diameter is preferably from 10 nm to 1 µm.

As the fluorescent particles, there may be mentioned, for example, particles of polystyrene, a styrene-butadiene copolymer, a styrene-acrylic acid copolymer or glass containing a fluorescent substance such as fluorescein, rhodamine and platinum cyanide. The colored particles are particles comprising an organic high molecular weight compound or an inorganic compound colored with various kinds of dyes or pigments such as, for example,
a material comprising one or a mixture of polystyrene, polymethyl acrylate, polyacrylamide, polypropylene, polycarbonate, glass and the like. The particle sizes of the above fluorescent particles and colored particles are preferably 10 nm to 1 µm.

The labelled substance and the genetic recombinant TP antigen can be linked by using known methods of forming a covalent bond or a non-covalent bond. As the binding method, there may be mentioned, for example, the glutaraldehyde method, the periodic acid method, the maleimide method, the pyridyl disulfide method and a method using various kinds of crosslinking agents (see, for example, "Protein, Nucleic acid and Enzyme", special edition, No. 31, pp. 37 to 45 (1985)). In the binding method using a crosslinking agent, for example, N-succinimidyl-4-maleimide butyric acid (GMBS), N-succinimidyl-6-maleimide hexanoic acid and N-succinimidyl-4-(N-maleimidomethyl)cyclohexane-1-carboxylic acid may be used as a crosslinking agent. For the method of covalent bonding, a functional group present in the genetic recombinant TP antigen can be used. After introducing a functional group such as a thiol group, an amino group, a carboxyl group and a hydroxy group into the genetic recombinant TP antigen, a labelled genetic recombinant TP antigen can be prepared by the above binding method. As the method by non-covalent bonding, there may be mentioned the physical adsorption method.

A method for labelling the genetic recombinant TP antigen by using a radioisotope can be carried out by, for example, using a Bolton Hunter reagent.

The amount of the labelled genetic recombinant TP antigen to be contained in the labelling reagent zone may be varied suitably depending on the method for dotting it to the matrix or incorporating it into a water-absorbable pad, the object to be tested and the amount of specimen used for the measurement, but it is generally about 0.01 µg to 5 µg in terms of dry weight. As the labelled genetic recombinant TP antigen, at least one antigen selected from genetic . recombinant TP antigens such as TP47, TP42, TP17 and TP15, which are labelled, may be used by adding it to the labelling reagent zone.

Further, the recombinant TP antigen may be a fusion antigen of syphilis treponema produced by fusing two or more antigens selected from the above-mentioned respective antigens, and includes, for example, TP15-17 antigen, TP15-42 antigen, TP15-47 antigen, TP17-42 antigen, TP17-47 antigen, TP42-47 antigen, TP15-17-42 antigen, TP15-42-47 antigen, TP15-17-47 antigen, TP17-42-47 antigen and TP15-17-42-47 antigen, or may be a fusion antigen of syphilis treponema in which the order of bonding of these antigens is changed.

### (Specimen dotting zone)

The specimen dotting zone can be provided without containing a reagent or the like, on the matrix downstream to the developing solution zone and upstream to the detection zone with regard to the transferring direction of the developing solution. Further, the specimen dotting zone can be provided downstream to the developing solution zone and upstream to the labelling reagent zone, or downstream to the labelling reagent zone and upstream to the detection zone with regard to the transferring direction of the developing solution. In the above device in which a water-absorbable pad is provided on the labelling reagent zone, it is preferred to provide a specimen dotting zone on the labelling reagent zone, and it is further preferred to provide the specimen dotting zone on said pad in order to increase the efficiency of the analysis.

In the device to which the pad is added, a large amount of a specimen solution can be dotted so that a minute amount of a component in a specimen can be analyzed with high detection sensitivity. The water-absorbing pad may be constituted by using, for example, one or a combination of materials comprising porous synthetic or natural high molecular weight compounds such as polyvinyl alcohol (PVA), a nonwoven fabric and cellulose. The size and thickness of the pad is not limited, but it is generally preferred to use a pad having a length and a width of about 3 mm to 10 mm and a thickness of about 0.5 mm to 4 mm in view of the efficiency of the measurement.

### (Detection zone)

The detection zone can be provided downstream to the specimen dotting zone with regard to the transferring direction of the developing solution. This zone can be provided by immobilizing the TP antigen to the matrix by a method of chemical bonding such as covalent bonding or physical adsorption. Also, the TP antigen may be bound to an insoluble carrier and be incorporated into the matrix. The insoluble carrier includes particles obtained by insolubilizing a mixture comprising gelatin, gum arabic and sodium hexamethaphosphate (Japanese Patent Publication No. 29223/1988), a polystyrene latex, red blood cells of various kinds of animals and a glass fiber. The insoluble carrier and the TP antigen can be bound by the above chemical bonding or physical adsorption. The detection zone may have any shape, but in order to improve detection sensitivity, it is preferred that the detection zone is formed in a linear shape so that it intersects the transferring direction of the developing solution perpendicularly.

The detection zone can be formed by immobilizing the above genetic recombinant TP antigens such as TP47, TP42, TP17 and TP15 to the matrix, respectively. Two or more detection zones can be provided by immobilizing the above antigens to different positions on the matrix, respectively. In the device in which two or more detection zones are provided, antibodies to the respective antigens can be detected separately. Alternatively, two or more genetic recombinant TP antigens may be mixed to prepare one detection zone. Furthermore, in the detection zone, the socalled cultured TP antigen (JP-A-14911/1988, JP-B-1,466,818) may be used which is obtained by decomposing cells of TP (Nicolle's strain) cultured in a living body such as a rabbit testicle according to a method known to a person skilled in the art and carrying out purification by using methods such as extraction and centrifugation in combination.

### (Developing solution zone)

The developing solution zone is a zone provided at one end of the matrix, to which a developing solution is supplied. Measurement can be started by dipping this zone in a container containing a developing solution at least in such an amount that the developing solution can reach to the developing solution-absorbing zone. The measurement can be also started by supplying a developing solution by adding a solution reservoir containing a developing solution to the developing solution zone and breaking the liquid reservoir to bring the developing solution into contact with the matrix. In the developing solution, a surfactant, a buffer and the like may be suitably contained. As a buffer solution there may be mentioned, for example, an acetate buffer solution, a borate buffer solution, a Tris-HCl buffer solution and a diethanolamine buffer solution. A water-absorbing filter paper or the like may be further provided on the developing solution zone depending on the supplying system of the developing solution.

### (Developing solution-absorbing zone)

The developing solution-absorbing zone is provided at the end of the matrix opposite to the developing solution zone. This zone is provided for absorbing the developing solution supplied to the matrix in order to carry out the analysis smoothly. The developing solution-absorbing zone can be also formed by using a long matrix. Development can be accelerated by providing a water-absorbing material on the matrix. As the water-absorbing material, a filter paper, a sponge or the like having high water retention property comprising a natural high molecular weight compound, a synthetic high molecular weight compound or the like can be used. By laminating the water-absorbing material on the matrix, an immunoassay device minimized in size can be prepared.

The device of the present invention can have various forms depending on the kind of the labelled substance. However, when an enzyme is used as the labelled substance, the device is an immunoassay device for the anti-TP antibody, in which a developing solution zone containing a substrate, a labelling reagent zone comprising a genetic recombinant TP antigen labelled with an enzyme, a detection zone in which a TP antigen is immobilized to a matrix which can transfer a solution by capillarity, a specimen dotting zone and a developing solution-absorbing zone are provided on the matrix.

In the device using the above enzyme as the labelled substance, various kinds of substrates can be used for detection. The substrate is used generally by adding it to the developing solution. As the substrate, there may be used the following kinds of coloring substrates, fluorescent substrates and light emission substrates depending on the enzyme.

### (a) Coloring substrate

for peroxidase: 2,2'-azino-bis(3-ethylbenzothiazolin-6-sulfonic acid) (ABTS), 3,3'-5,5'-tetramethylbenzidine (TMB) or diaminobenzidine (DAB) in combination with hydrogen peroxide;
for alkaline phosphatase: 5-bromo-4-chloro-3-indolylphosphoric acid (BCIP);

### (b) Fluorescent substrate

for alkaline phosphatase: 4-methylumbelliphenylphosphate (4MUP);
for β-D-galactosidase: 4-methylumbelliphenyl-β-D-galactoside (4MUG);

### (c) Light emission substrate

for alkaline phosphatase: disodium 3-(2'-spiroadamantan)-4-methoxy-4-(3"-phosphoryloxy)phenyl-1,2-dioxetane (AMPPD);
for β-D-galactosidase: 3-(2'-spiroadamantan)-4-methoxy-4-(3''-β-D-galactopyranosyl)phenyl-1,2-dioxetane (AMGPD); and
for peroxidase: luminol or isoluminol in combination of hydrogen peroxide.

The above substrate may be provided as a substrate zone on the matrix. The substrate zone provided on the matrix can be formed generally by dissolving the substrate in a solution, adding the resulting solution to the matrix and then drying the solution.

Further, as an embodiment of the device of the present invention, there may be mentioned an immunoassay device for the anti-TP antibody, in which a specimen developing solution zone is provided on the matrix for adding a developing solution containing a specimen and a genetic recombinant syphilis treponema antigen labelled with a radioisotope, a latex, metal colloid particles, fluorescent particles or colored particles, as well as a detection zone in which a TP antigen is immobilized to a matrix which can transfer a solution by capillarity and a developing solution-absorbing zone.

This assay device is different from the above device in that a specimen and a genetic recombinant TP antigen labelled with a radioisotope, a latex, metal colloid particles, fluorescent particles or colored particles are added to the specimen developing solution zone. The specimen developing solution zone can be formed by adding a solution containing the above labelled genetic recombinant TP antigen and a solution containing a specimen, respectively, or by adding a mixed solution containing the labelled genetic recombinant TP antigen and a specimen. To the developing solution, a developing solution containing the above surfactant, buffer or the like may be added, if desired. The labelled genetic recombinant TP antigen to be added to the specimen developing solution zone is a genetic recombinant TP antigen labelled with a radioisotope, a latex, colloid particles or colored particles and may be selected from the labelled genetic recombinant TP antigens used in the above assay device. Further, the detection zone and the developing solution-absorbing zone of the assay device may be the same as those of the above assay device.

### (Method of use)

By using the assay device of the present invention, the anti-TP antibody in various kinds of specimens can be analyzed. When an analysis is carried out using the device, a specimen is first supplied to the matrix of the device and then developed with a developing solution. The developing solution reaches the developing solution-absorbing zone by capillarity, and components in the specimen and the labelled genetic recombinant TP antigen which are not bound to the detection zone are absorbed to complete development. After development is finished, the labelled substance which is immobilized to the detection zone in an amount depending on the anti-TP antibody in the specimen solution is directly or indirectly detected to determine the presence or absence of anti-TP antibodies.
The detection can be carried out by visual observation with eyes or by using a measurement device such as a scintillation counter, a colorimeter, a fluorophotometer, a photocounter and a light-sensitive film depending on the labelled substance. In the analysis, a method of using, for example, an enzyme as the labelled substance and measuring visually with the eyes the presence or absence of a dye formed by a color emission substrate qualitatively is simple and easy. By this method, semiquantitative analysis can be carried out by using a color chart corresponding to the concentration of the anti-TP antibody.

The matrix may be laminated on and fixed to a support member such as a plastic, a metal and a paper depending on the kind of the labelled substance. Further, by fixing the matrix to a case made of plastic or the like, providing a solution reservoir containing the developing solution on the developing solution zone and laying covers each having a hole on the above respective zone portions, a device which can be handled easily can be constituted. In the immunoassay device of the present invention, the specimen is not particularly limited, and the device can be suitably used for detecting the anti-TP antibody in various kinds of body fluids such as serum, plasma, whole blood and urine.

### EXAMPLES

The present invention is descried in detail by referring to the following Examples. As a matter of course, the present invention is not limited to the Examples described below.

### Example 1

At both ends of a membrane portion comprising a nitrocellulose membrane having a size of 5 mm x 50 mm, a developing solution-supplying portion and a water absorption portion each comprising a water-absorbing nonwoven fabric were provided, respectively. An anti-HBs polyclonal antibody was dotted to a part of the membrane portion, which was near the water absorption portion, and dried to give a detection line. Then, after the membrane was blocked, in the device A, a labelled substance pad comprising a water-absorbable nonwoven fabric which was a square having a side length of 5 mm and a thickness of 1 mm was provided on the membrane. 10 µl of a solution containing an alkaline phosphatase-labelled anti-HBs monoclonal antibody at a concentration of 12.5 µg/ml was dotted to the labelled substance pad and dried to prepare an assay device. On the other hand, in the device B, without providing a labelled substance pad, 5 µl of a solution containing an alkaline phosphatase-labelled anti-HBs monoclonal antibody at a concentration of 25 µg/ml was directly dotted to the membrane and dried to prepare an assay device.

As specimen solutions, standard solutions containing an HBs antigen at a concentration of 0 to 90 ng/ml and an HBs-negative serum or HBs-positive sera diluted to 32 to 256 times were used. In the device A, 25 µl of these specimen solutions were added to the labelled substance pad, respectively, and in the device B, 5 µl of the specimen solutions were added to the labelled substance dotting portion, respectively. On the other hand, 200 µl of a developing solution was added to each developing solution-supplying portion. The developing solution contained toluidine 5-bromo-4-chloro-3-indolylphosphate as a substrate, at a concentration of 1.5 mg/ml. Coloring at the detection line 15 minutes, 30 minutes and 60 minutes after addition of the developing solution was observed. The results are shown in the following Table 1.

**Table 1**

| Judgment time | | 15 minutes | | 30 minutes | | 60 minutes | |
|---|---|---|---|---|---|---|---|
| Assay device | | A | B | A | B | A | B |
| Concentration | 0 | - | - | - | - | - | - |
| of antigen in | 5 | - | - | - | - | - | - |
| standard solu- | 10 | - | - | + | - | + | - |
| tion | 30 | + | - | + | - | + | + |
| (ng/ml) | 90 | + | ± | + | + | + | + |
| Negative serum | | - | - | - | - | - | - |
| Positive serum diluted to 256 times | | - | - | ± | - | + | ± |
| Positive serum diluted to 128 times | | ± | - | + | - | + | + |
| Positive serum diluted to 64 times | | + | - | + | ± | + | + |
| Positive serum diluted to 32 times | | + | + | + | + | + | + |

In Table 1, "-" means that coloring was not observed on the detection line, "+" means that coloring was clearly observed on the detection line; and "±" means that coloring was not clearly observed, but was barely observed.

### Example 2

To a membrane comprising a nitrocellulose membrane, a developing solution pad portion and a water-absorption pad portion were provided, respectively, in the same manner as in Example 1. An HBs antigen (0.75 µg) was dotted to a part of the membrane portion, which was the same dotting portion as the anti-HBs polyclonal antibody, and dried to give a detection line. Then, after the membrane was blocked, in the device C, a labelled substance pad comprising a water-absorbable nonwoven fabric which was a square having a size of 6 mm x 10 mm and a thickness of 0.5 mm was provided on the membrane. 5 µl (25 ng) of an alkaline phosphatase-labelled anti-HBs antigen was dotted to the labelled substance pad and dried to prepare an assay device.

In the device D, without providing a labelled substance pad, the above-mentioned labelled HBs antigen was dotted to the membrane and dried to prepare an assay device.

As specimen solutions, standard solutions containing an anti-HBs antibody at a concentration of 0 to 64.5 mIU/ml were used. In the above-mentioned assay devices C and D, developments were carried out. Coloring at the detection line 11 minutes, 15 minutes and 20 minutes after addition of the developing solution was observed. The results are shown in the following Table 2.

**Table 2**

| Judgment time | | 11 minutes | | 15 minutes | | 20 minutes | |
|---|---|---|---|---|---|---|---|
| Assay device | | C | D | C | D | C | D |
| Concentration of antibody in standard solution (mIU/ml) | 0 | - | - | - | - | - | - |
| | 15.6 | - | - | + | - | + | + |
| | 26.6 | - | - | + | - | + | + |
| | 64.5 | + | - | + | + | + | + |
| "-" and "+" mean the same judgment standard as in Table 1. | | | | | | | |

### Example 3

At both ends of a membrane portion comprising a nitrocellulose membrane having a size of 5 mm x 50 mm, a developing solution pad portion and a water-absorption pad portion each comprising a water-absorbing nonwoven fabric were provided, respectively. An anti-hemoglobin polyclonal antibody was dotted to a part of the membrane portion, which was near the water-absorption pad portion, and dried to give a detection line. Then, after the membrane was blocked, a labelled substance pad comprising a water-absorbing nonwoven fabric which was a square having a side length of 5 mm and a thickness of 1 mm was provided on the membrane. 10 µl of a solution containing an alkaline phosphatase-labelled anti-hemoglobin polyclonal antibody at a concentration of 15 µg/ml was dotted to the labelled substance pad and dried. Further, in the device of Example 1, a substrate-containing portion was provided on the membrane portion between the labelled substance pad and the developing solution pad portion to prepare an assay device. The substrate-containing portion was formed by repeating an operation of dotting 5 µl of a solution containing 20 mg/ml of sodium 5-bromo-4-chloro-3-indolyl-phosphate (BCIP, Na) and drying the solution three times.

### Example 4

On the other hand, in the device of Example 4, a substrate-containing portion was not provided.

25 µl of a sample containing 5, 10 or 20 ng/ml of hemoglobin were dotted to the labelled substance pad, and then 200 µl of a developing solution were added to the developing solution pad to start the measurement.

As the developing solution, a solution comprising a 0.1 M 2,2'-iminodiethanol-HCl buffer solution (pH 10), 1 mM MgCl₂ and 0.05 % Tween 20 (trade name, produced by Atlas Powder Co.) was used. In Comparative example 1, a solution obtained by adding BCIP, Na to the developing solution used in Example 3 at a concentration of 0.3 mg/ml was used as the developing solution. The judgment time was 8 minutes after the developing solution was added, and judgment was further carried out 10 minutes and 30 minutes after the starting point. The respective tests were carried out twice, respectively. The results are shown in Table 3 and Table 4.

**Table 3**

| Results of Example 3 | | | |
|---|---|---|---|
| Judgment time (min) | Hemoglobin concentration | | |
| | 20 ng/ml | 10 ng/ml | 5 ng/ml |
| 8 | ++ | -- | -- |
| 10 | ++ | ±- | -- |
| 30 | ++ | ±- | -- |

**Table 4**

| Results of Example 4 | | | |
|---|---|---|---|
| Judgment time (min) | Hemoglobin concentration | | |
| | 20 ng/ml | 10 ng/ml | 5 ng/ml |
| 8 | -- | -- | -- |
| 10 | ±± | -- | -- |
| 30 | ++ | ++ | ++ |

It can be seen that when the device of Example 4 was used, the judgment results were reversed from negative to positive with a lapse of time, and when the device of Example 3 was used, there was almost no change in judgment from negative to positive. Thus, when a reaction is terminated after a certain reaction time and the measurement is not carried out before the lapse of some time, it is preferred to use the device of Example 3.

### Comparative example 1

A conventional assay device using a color latex as shown in Fig. 4 was prepared. A detection line was prepared in the same manner as in Example 3. 100 µl of the same specimen solution as in Example 3 was added to a sample dotting position to start the measurement. The judgment time was 8 minutes after the sample was added, and judgment was further carried out 10 minutes and 30 minutes after the starting point. The results are shown in Table 5.

**Table 5**

| Results of Comparative example 1 | | | |
|---|---|---|---|
| Judgment time (min) | Hemoglobin concentration | | |
| | 20 ng/ml | 10 ng/ml | 5 ng/ml |
| 8 | ±± | -- | -- |
| 10 | ±± | -- | -- |
| 30 | ++ | ++ | ±± |

From Table 5, it can be seen that even in the conventional method using a color latex, the judgment results were reversed from negative to positive.

### Example 5 Preparation of a genetic recombinant TP17 antigen labelled with alkaline phosphatase

200 nmol of 2-iminothiolan was added to 0.12 mg of a genetic recombinant TP17 antigen to which GST was bound, prepared according to the method described in unexamined Japanese Patent application with Publication No. 287017/1995, and the mixture was left to stand at 30 °C for 60 minutes to obtain a TP antigen into which thiol was introduced. Next, 300 nmol of N-succinimidyl-4-maleimide butyric acid (GMBS) was added to 3 mg of alkaline phosphatase, and the mixture was left to stand at 30 °C for 60 minutes to obtain alkaline phosphatase into which maleimide was introduced. Thereafter, 100 µg of the TP antigen into which thiol was introduced and 2.5 mg of alkaline phosphatase into which maleimide was introduced were mixed, and the mixture was left to react at 4 °C overnight. By using a gel filtration column, unreacted antigen and enzyme were removed to obtain an alkaline phosphatase-labelled genetic recombinant TP17 antigen.

### Example 6 Device for analyzing an anti-TP17 antibody

As shown in Fig. 2, at a position of 15 mm from the top end of a matrix 1 which was a cellulose film (produced by Millipore Co.) having a width of 5 mm and a length of 50 mm, the same genetic recombinant TP17 antigen used in Example 5 was dotted in a line by a coating device and then dried to prepare a detection zone 5. 20 µl of a solution containing the alkaline phosphatase-labelled genetic recombinant TP17 antigen prepared in Example 5 were dotted to a polyvinyl alcohol (PVA) sheet cut so as to have a width of 5 mm and a length of 5 mm and then dried to prepare a pad. The pad was placed at a position of 25 mm from the top end of the matrix 1 to prepare a labelling reagent zone 4 and a specimen dotting zone 3. At a position of 10 mm from the bottom end of the matrix 1, a filter paper (produced by Millipore Co.) having a width of 5 mm and a length of 20 mm was provided to prepare a developing solution zone 2. Further, as a developing solution-absorbing zone 6, a filter paper having a width of 10 mm, a length of 20 mm and a thickness of about 1 mm was provided at a position of 10 mm from the top end of the matrix 1 to obtain a device for analyzing an anti-TP17 antibody.

### Example 7 Measurement of an anti-TP17 antibody

An anti-TP antibody was analyzed by using the assay device prepared in Example 6. In the first place, 15 µl of a specimen were dotted to the specimen dotting zone 3, 200 µl of a solution containing 0.3 % 5-bromo-4-chloro-3-indolylphosphoric acid (BCIP) were added dropwise to the developing solution zone 2 to effect absorption and development. After 15 minutes, the coloring degree of the detection zone 5 was judged visually by eye. 89 human serum specimens were measured, and the results are shown in Table 6. A case where coloring was observed was judged as positive, and a case where coloring was not observed was judged as negative.

Also, with respect to the same specimen, a diluted series was prepared and measured by using a conventional indirect agglutination immunoassay reagent Serodia-TP-PA (registered trade name, produced by Fujirebio Inc.) for the anti-TP antibody. The results are shown in Table 6.

### Example 8 Measurement of an anti-TP47 antibody

By using a recombinant TP47 antigen prepared according to the method of Norgard et al. (INFECTION AND IMMUNITY, Vol. 61, pp. 1202 to 1210, 1993) and then purified, an alkaline phosphatase-labelled recombinant TP47 antigen was prepared and further a device for analyzing an anti-TP47 antibody was obtained according to the methods of Examples 5 and 6. By using the device, specimens shown in Table 6 were measured. The results of Examples 7 and 8 are shown in Table 6. When the specimens of Examples 7 and 8 giving a positive result in at least one of the devices were judged as positive, the same results as those obtained by the indirect agglutination method were obtained in all of the specimens.

**Table 6**

| Indirect agglutination method (dilution ratio) | Assay device for TP17 antibody | Assay device for TP17 and TP47 antibodies (positive rate; %) |
|---|---|---|
| | Positive specimen/tested specimen (positive rate; %) | |
| Negative specimen (>1:80) | 0/28 (0) | (0) |
| Positive specimen (1:80) | 2/2 (100) | (100) |
| Positive specimen (1:160) | 3/3 (100) | (100) |
| Positive specimen (1:320) | 15/15 (100) | (100) |
| Positive specimen (1:640) | 5/6 (83.3) | (100) |
| Positive specimen (1:1280) | 9/9 (100) | (100) |
| Positive specimen (1:2560) | 8/8 (100) | (100) |
| Positive specimen (1:2560<) | 18/18 (100) | (100) |

### Example 9 Device for analyzing an anti-TP17 antibody and an anti-TP47 antibody

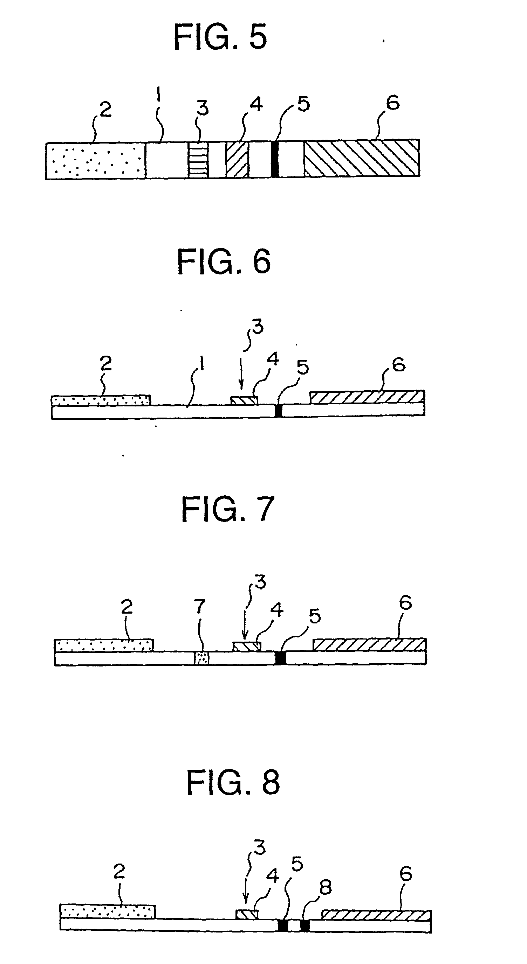

As shown in Fig. 8, at a position of 12 mm from the top end of a matrix 1 which was a cellulose film (produced by Millipore Co.) having a width of 5 mm and a length of 50 mm, a recombinant TP17 antigen was dotted in a line by a coating device, and at a position of 17 mm from the top end of the matrix 1, a genetic recombinant TP47 antigen was dotted in a line by a coating device. The antigens coated were dried to prepare detection zones 5 and 8. 20 µl of a mixed solution containing the alkaline phosphatase-labelled recombinant TP17 antigen prepared in Example 5 and the alkaline phosphatase-labelled recombinant TP47 antigen prepared in Example 7were dotted to a PVA sheet cut so as to have a width of 5 mm and a length of 5 mm and then dried to prepare a conjugate pad. The conjugate pad was placed at a position of 25 mm from the top end of the matrix 1 to prepare a labelling reagent zone 4.

Paper filters of a developing solution zone 2 and a developing solution-absorbing zone 6 were provided in the same manner as in Example 6 to prepare a device for analyzing an anti-TP17 antibody and an anti-TP47 antibody.

### Example 10 Measurement of an anti-TP17 antibody and an anti-TP47 antibody

With respect to the specimens giving a positive result by the indirect agglutination immunoassay method, anti-TP antibodies were measured by using the device prepared in Example 9. In the first place, 15 µl of a specimen were added to the specimen dotting zone 3 provided on the labelling reagent zone 4, and 200 µl of a solution containing 0.3 % BCIP were added dropwise to the developing solution zone 2 to effect absorption and development. After 15 minutes, the coloring degrees of the detection zones 5 and 8 were judged visually by eye, A case where coloring was observed was judged as positive, and a case where coloring was not observed was judged as negative.

The specimens judged as positive or negative by the above indirect agglutination immunoassay reagent were measured by the assay device of Example 9. The results are shown in Table 7. Further, a primary stage syphilis-infected specimen, a secondary stage infected specimen and a tardive stage infected specimen which were known were measured by using the indirect agglutination immunoassay and the assay device of Example 8, respectively. The results are shown in Table 8.

**Table 7**

| Specimen | Indirect agglutination method | Assay device for analyzing TP17 and TP47 antibodies | |
|---|---|---|---|
| | | TP17 | TP47 |
| 1 | + | + | + |
| 2 | + | + | - |
| 3 | + | - | + |
| 4 | - | - | - |
| +: positive judgment, -: negative judgment | | | |

**Table 8**

| Specimen | Indirect agglutination method | Assay device for analyzing TP17 and TP47 antibodies | |
|---|---|---|---|
| | | TP17 | TP47 |
| Primary stage syphilis-infected specimen | + | ± | + |
| Secondary stage syphilis-infected specimen | + | + | ± |
| Tardive stage syphilis-infected specimen | + | + | ± |
| +: positive judgment, ±: weak positive judgment | | | |

### Example 11 Preparation of alkaline phosphatase-labelled TP15-17 antigen

A genetic recombinant TP15-17 antigen in which a TP15 antigen and a TP17 antigen were fused was prepared, and according to the same manner as described in Example 5, an alkaline phosphatase-labelled TP15-17 antigen was obtained.

### Example 12 Device for analyzing an anti-TP15-17 antibody

According to the same manner as in Example 6, the alkaline phosphatase-labelled TP15-17 antigen prepared in Example 11 and the genetic recombinant TP15-17 antigen were dotted onto a matrix 1 to prepare a device for analyzing an anti-TP15-17 antibody.

### Example 13 Device for analyzing an anti-TP15-17 antibody

By using the analyzing device prepared in Example 12, analysis of an anti-TP antibody was carried out for 5 specimens of human sera according to the method of Example 7. The results are shown in Table 9.

Further, with regard to the same specimens, the results according to the above-mentioned indirect agglutination immunoassay reagent and anti-TP 47 antibody analyses are also shown in Table 9.

**Table 9**

| Specimen | Indirect agglutination immunoassay reagent | TP15-17 antibody assay device | TP47 antibody assay device |
|---|---|---|---|
| 1 | + | + | + |
| 2 | + | + | + |
| 3 | +/- | + | - |
| 4 | +/- | + | - |
| 5 | - | - | - |
| +: positive judgment, +/-: judgment reserved, -: negative judgment | | | |

The present invention can provide an immunoassay device in which detection sensitivity is higher than that of a conventional immunoassay device.

The present invention also provides an immunoassay device in which a judgment result at the time of judgment is not reversed from negative to positive with a lapse of time, and an immunoassay method using the same.

The present invention further provides a method for analyzing the anti-TP antibody in a specimen simply and easily with a high sensitivity. In this method, for example, the anti-TP antibody in serum can be analyzed within about 15 minutes to obtain a result. The prozone phenomenon observed at the time of measuring a specimen having a high antibody value of the anti-TP antibody was not observed in the assay method of the present invention. Further, when the method of the present invention is used, a plurality of the anti-TP antibodies in a specimen can be analyzed separately so that the stage of infection can be estimated, and the method is also useful for treating syphilis.

## Claims

1. An immunoassay device for detecting an antigen or an antibody, the device comprising an elongate matrix (1, 10) having disposed therealong in order between the matrix ends:
(i) a zone (4, 6, 17) containing a labelled antibody or antigen which is capable of reacting with the antigen or antibody to be detected; and
(ii) at least one detection zone (5, 8, 18) where an antibody or antigen, capable of reacting with the antigen or antibody to be detected, is immobilized;
**characterized in that** a zone (iii) for supplying the developing solution is provided at an upstream side of the developing solution transporting direction of the zone (i), a substance for labelling the antibody or antigen contained in the zone (ii) is an enzyme, a substrate for said enzyme is provided within zone (iii) and/or between zone (i) and zone (iii), and a zone (iv) for dotting a specimen is provided on the zone (i).

2. Device according to Claim 1, wherein the zone (i) is made from a pad comprising an absorbing material.

3. Device according to Claim 1 or 2, wherein the zone (i) comprises a genetic recombinant syphilis treponema antigen labelled with an enzyme, and the zone (ii) comprises a syphilis treponema antigen immobilized to the matrix.

4. Device according to Claim 3, wherein the syphilis treponema antigen of the detection zone is a genetic recombinant syphilis treponema antigen.

5. Device according to Claim 3 or 4, wherein the genetic recombinant syphilis treponema antigen is at least one antigen selected from the group consisting of a TP15 antigen having a molecular weight of 15 kg/mol (15kDa), a TP17 antigen having a molecular weight of 17 kg/mol (17 kDa), a TP42 antigen having a molecular weight of 42 kg/mol (42 kDa), a TP47 antigen having a molecular weight of 47 kg/mol (47 kDa) and a fusion antigen of syphilis treponema in which two or more above syphilis treponema antigens are fused.

6. Use of the device according to Claims 1 to 5 in an immunoassay method.

7. Use according to Claim 6 for the detection of anti-syphilis treponema antibodies.

8. Immunoassay method, comprising the steps of:
dotting a specimen solution on the zone (i) of the device according to any of Claims 1 to 5;
after sufficiently making the enzyme-labelled antibody or antigen react with the antigen or antibody to be detected, supplying a developing solution to the zone (iii) of said device; and
detecting the presence or absence of the antigen or antibody to be detected by observing the reaction of the enzyme with its substrate in the zone (ii).

9. Immunoassay method according to Claim 8, wherein anti-syphilis treponema antibodies are detected.

10. An immunoassay device for detecting anti-syphilis treponema antibodies, the device comprising an elongate matrix (1, 10) having disposed therealong in order between the matrix's ends:
(ia) a zone (2, 12) for supplying a developing solution to the matrix; and
(iia) at least one detection zone (5, 8, 18) where a syphilis treponema antigen is immobilised to the matrix; so that when the developing solution or the developing solution containing the specimen to be analyzed and genetic recombinant syphilis treponema antigen labelled with a radioisotope, a latex, metal colloid particles, fluorescent particles or coloured particles is supplied to the zone (ia), it is transported by capillary action along the matrix so that it contacts with the zone (iia).

11. Device according to Claim 10, wherein the developing solution supplied to the zone (ia) does not contain genetic recombinant syphilis treponema antigen labelled with a radioisotope, a latex, metal colloid particles, fluorescent particles or coloured particles, and a zone (iiia) is provided on the matrix between zones (ia) and (iia), which contains genetic recombinant syphilis treponema antigen labelled with a radioisotope, a latex, metal colloid particles, fluorescent particles or coloured particles.

12. Device according to Claim 11, wherein zone (iiia) comprises a water-absorbing pad containing the genetic recombinant syphilis treponema antigen labelled with a radioisotope, a latex, metal colloid particles, fluorescent particles or coloured particles.

13. An immunoassay method for detecting an anti-syphilis treponema antibody, which comprises the steps of:
applying a developing solution containing the specimen to be analyzed and a genetic recombinant syphilis treponema antigen labelled with a radioisotope, a latex, metal colloid particles, fluorescent particles or coloured particles to zone (ia) of the device according to Claim 10; and detecting the presence or absence of anti-syphilis treponema antibodies by detecting the labelled antigen trapped in the zone (iia), the amount of which depends on the amount of anti-syphilis treponema antibodies to be detected.

14. An immunoassay method for detecting an anti-syphilis treponema antibody, which comprises steps of:
dotting a specimen solution to zone (iiia) of the device according to Claim 11 or 12;
supplying a developing solution to the zone (ia) of said device; and
detecting the presence or absence of anti-syphilis treponema antibodies by detecting the labelled antigen trapped in the zone (iia), the amount of which depends on the amount of anti-syphilis treponema antibodies to be detected.

## Patentansprüche

1. Vorrichtung für ein Immunassay zum Nachweis eines Antigens oder eines Antikörpers, wobei die Vorrichtung eine langgezogene Matrix (1, 10) umfasst und zwischen den Matrixenden:
(i) eine Zone (4, 6, 17), die einen markierten Antikörper oder ein markiertes Antigen enthält, der/das mit dem nachzuweisenden Antigen oder Antikörper reagieren kann, und
(ii) mindestens eine Nachweiszone (5, 8, 18), an die ein Antikörper oder ein Antigen, der/das mit dem nachzuweisenden Antigen oder Antikörper reagieren kann, immobilisiert ist,
liegen,
**dadurch gekennzeichnet, dass** eine Zone (iii) zum Auftragen der Entwicklungslösung an der stromaufwärts liegenden Seite der Transportrichtung der Zone (i) der Entwicklungslösung bereitgestellt ist, die Substanz zum Markieren des Antikörpers oder Antigens, der/das in der Zone (ii) enthalten ist, ein Enzym ist, ein Substrat für dieses Enzym in der Zone (iii) und/oder zwischen der Zone (i) und der Zone (iii) bereitgestellt ist und eine Zone (iv) zum Auftragen einer Probe auf der Zone (i) vorliegt.

2. Vorrichtung nach Anspruch 1, wobei die Zone (i) aus einer Auflage besteht, die ein absorbierendes Material umfasst.

3. Vorrichtung nach Anspruch 1 oder 2, wobei die Zone (i) ein genetisch rekombinantes Syphilis treponema-Antigen, das mit einem Enzym markiert ist, umfasst, und die Zone (ii) ein Syphilis treponema-Antigen, das an der Matrix immobilisiert ist, umfasst.

4. Vorrichtung nach Anspruch 3, wobei das Syphilis treponema-Antigen der Nachweiszone ein genetisch rekombinantes Syphilis treponema-Antigen ist.

5. Vorrichtung nach Anspruch 3 oder 4, wobei das Syphilis treponema-Antigen mindestens ein Antigen ist, ausgewählt aus der Gruppe, bestehend aus einem TP15 Antigen mit einem Molekulargewicht von 15 kg/mol (15 kDa), einem TP17 Antigen mit einem Molekulargewicht von 17 kg/mol (17 kDa), einem TP42 Antigen mit einem Molekulargewicht von 42 kg/mol (42 kDa), einem TP47 Antigen mit einem Molekulargewicht von 47 kg/mol (47 kDa) und einem Fusionsantigen von Syphilis treponema, bei dem zwei oder mehrere der obigen Syphilis treponema-Antigene verbunden sind.

6. Verwendung der Vorrichtung nach Ansprüchen 1 bis 5 bei einem Immunassayverfahren.

7. Verwendung nach Anspruch 6 zum Nachweis von Anti-Syphilis treponema-Antikörpern.

8. Immunassayverfahren, umfassend die Schritte:
Aufbringen einer Probenlösung in Form eines Punktes auf die Zone (i) der Vorrichtung nach einem der Ansprüche 1 bis 5, und nachdem gewährleistet ist, dass der Enzymmarkierte Antikörper oder Antigen mit dem nachzuweisenden Antigen oder Antikörper ausreichend reagiert hat,
Auftragen einer Entwicklungslösung auf der Zone (iii) der Vorrichtung und
Nachweisen der Gegenwart oder Abwesenheit des nachzuweisenden Antigens oder Antikörpers durch Feststellen der Reaktion des Enzyms mit seinem Substrat in der Zone (ii).

9. Immunassayverfahren nach Anspruch 8, wobei Anti-Syphilis treponema-Antikörper nachgewiesen werden.

10. Immunoassayvorrichtung zum Nachweisen von Anti-Syphilis treponema-Antikörpern, wobei die Vorrichtung eine langgezogene Matrix umfasst (1, 10) und zwischen den Enden der Matrix:
(ia) eine Zone (2, 12) zum Auftragen einer Entwicklungslösung auf die Matrix und,
(iia)mindestens eine Nachweiszone (5, 8, 18), wobei ein Syphilis treponema-Antigen an der Matrix immobilisiert ist, so dass beim Auftragen der Entwicklungslösung oder der Entwicklungslösung, die die zu analysierende Probe und das genetisch rekombinante Syphilis treponema-Antigen, das mit einem Radioisotop, einer Latex, Metallcolloidpartikeln, fluoreszierenden Partikeln oder gefärbten Partikeln markiert ist, enthält, auf die Zone (ia), diese über Kapillarwirkung entlang der Matrix transportiert wird, so dass sie in Kontakt mit der Zone (iia) gebracht wird.

11. Vorrichtung nach Anspruch 10, wobei die Entwicklungslösung, die auf die Zone (ia) aufgetragen wird, nicht das genetisch rekombinante Syphilis triponema-Antigen enthält, das mit einem Radioisotop, einer Latex, Metallcolloidpartikeln, fluoreszierenden Partikeln oder gefärbten Partikeln markiert ist, und eine Zone (iiia), auf der Matrix zwischen den Zonen (ia) und (iia) bereitgestellt wird, die das genetisch rekombinante Syphilis triponema-Antigen enthält, das mit einem Radioisotop, einer Latex, Metallcolloidpartikeln, fluoreszierenden Partikeln oder gefärbten Partikeln markiert ist.

12. Vorrichtung nach Anspruch 11, wobei die Zone (iiia) eine wasserabsorbierende Auflage umfasst, die das genetisch rekombinante Syphilis triponema-Antigen enthält, das mit einem Radioisotop, einer Latex, Metallcolloidpartikeln, fluoreszierenden Partikeln oder gefärbten Partikeln markiert ist.

13. Immunassayverfahren zum Nachweisen eines Anti-Syphilis treponema-Antikörpers, umfassend die Schritte:
Auftragen einer Entwicklungslösung, die die zu analysierende Probe und ein genetisch rekombinantes Syphilis triponema-Antigen, das mit einem Radioisotop, einer Latex, Metallcolloidpartikeln, fluoreszierenden Partikeln oder gefärbten Partikeln markiert ist, enthält, auf die Zone (ia) der Vorrichtung nach Anspruch 10 und
Nachweisen der Gegenwart oder Abwesenheit von Anti-Syphilis treponema-Antikörpern, indem das markierte Antigen, das in der Zone (iia) festgehalten ist, nachgewiesen wird, wobei dessen Menge von der Menge der nachzuweisenden Anti-Syphilis treponema-Antikörper abhängt.

14. Immunassayverfahren zum Nachweisen eines Anti-Syphilis treponema-Antikörpers, umfassend die Schritte:
Aufbringen einer Probenlösung in Form eines kleinen Punktes auf die Zone (iiia) der Vorrichtung nach Anspruch 11 oder 12,
Auftragen einer Entwicklunsgslösung auf der Zone (ia) der Vorrichtung und
Nachweisen der Gegenwart oder Abwesenheit von Anti-Syphilis treponema-Antikörpern durch Nachweisen des markierten Antigens, das in der Zone (iia) festgehalten ist, wobei dessen Menge von der nachzuweisenden Menge der Anti-Syphilis treponema-Antikörper abhängt.

## Revendications

1. Dispositif d'analyse immunologique pour détecter un antigène ou un anticorps, le dispositif comprenant une matrice allongée (1, 10), le long de laquelle sont disposées d'une façon ordonnée entre les extrémités de la matrice:
(i) une zone (4, 6, 17) contenant un anticorps ou antigène marqué qui est capable de réagir avec l'antigène ou l'anticorps à détecter; et
(ii) au moins une zone de détection (5, 8, 18) où un anticorps ou antigène, capable de réagir avec
l'antigène ou l'anticorps à détecter, est immobilisé ; **caractérisé en ce qu'**une zone (iii) en vue de fournir la solution révélatrice est prévue au niveau d'un côté de la zone (i) en amont par rapport à la direction de transport de la solution révélatrice, une substance pour marquer l'anticorps ou l'antigène contenu dans la zone (ii) est une enzyme, un substrat pour ladite enzyme est prévu à l'intérieur de la zone (iii) et/ou entre la zone (i) et la zone (iii) et une zone (iv) pour le dépôt ponctuel d'un échantillon est prévue dans la zone (i).

2. Dispositif selon la revendication 1, dans lequel la zone (i) est fabriquée à partir d'un tampon comprenant une matière absorbante.

3. Dispositif selon la revendication 1 ou 2, dans lequel la zone (i) comprend un antigène recombinant génétique de tréponème de la syphilis, marqué avec une enzyme, et la zone (ii) comprend un antigène de tréponème de la syphilis, immobilisé sur la matrice.

4. Dispositif selon la revendication 3, dans lequel l'antigène de tréponème de la syphilis de la zone de détection est un antigène recombinant génétique de tréponème de la syphilis.

5. Dispositif selon la revendication 3 ou 4, dans lequel l'antigène recombinant génétique de tréponème de la syphilis est au moins un antigène choisi dans le groupe formé par un antigène TP15 ayant une masse moléculaire de 15 kg/mol (15 kDa), un antigène TP17 ayant une masse moléculaire de 17 kg/mol (17 kDa), un antigène TP42 ayant une masse moléculaire de 42 kg/mol (42 kDa), un antigène TP47 ayant une masse moléculaire de 47 kg/mol (47 kDa) et un antigène de fusion du tréponème de la syphilis dans lequel deux ou plusieurs antigènes du tréponème de la syphilis ci-dessus sont fusionnés.

6. Utilisation du dispositif selon les revendications 1 à 5, dans un procédé d'analyse immunologique.

7. Utilisation selon la revendication 6 pour la détection d'anticorps anti-tréponème de la syphilis.

8. Procédé d'analyse immunologique, comprenant les étapes consistant :
à déposer ponctuellement une solution d'échantillon sur la zone (i) du dispositif selon l'une quelconque des revendications 1 à 5;
à fournir une solution révélatrice à la zone (iii) dudit dispositif après avoir fait suffisamment réagir l'anticorps ou antigène marqué d'une enzyme, avec l'antigène ou l'anticorps à détecter; et
à détecter la présence ou l'absence de l'antigène ou de l'anticorps à détecter en observant la réaction de l'enzyme avec son substrat dans la zone (ii).

9. Procédé d'analyse immunologique selon la revendication 8, dans lequel des anticorps anti-tréponème de la syphilis sont détectés.

10. Dispositif d'analyse immunologique pour détecter des anticorps anti-tréponème de la syphilis, le dispositif comprenant une matrice allongée (1, 10), le long de laquelle sont disposées d'une façon ordonnée entre les extrémités de la matrice:
(ia) une zone (2, 12) pour fournir une solution révélatrice à la matrice; et
(iia) au moins une zone de détection (5, 8, 18) où un antigène de tréponème de la syphilis est immobilisé sur la matrice, de sorte que lorsque la solution révélatrice ou la solution révélatrice contenant l'échantillon à analyser et l'antigène recombinant génétique de tréponème de la syphilis, marqué d'un radio-isotope, d'un latex, de particules métalliques colloïdales, de particules fluorescentes ou de particules colorées, est fournie à la zone (ia), elle est transportée par un effet capillaire le long de la matrice de sorte qu'elle vient en contact avec la zone (iia).

11. Dispositif selon la revendication 10, dans lequel la solution révélatrice fournie à la zone (ia) ne contient pas d'antigène recombinant génétique de tréponème de la syphilis, marqué d'un radio-isotope, d'un latex, de particules métalliques colloïdales, de particules fluorescentes ou de particules colorées, et une zone (iiia) est prévue sur la matrice entre les zones (ia) et (iia), qui contient un antigène recombinant génétique de tréponème de la syphilis, marqué d'un radio-isotope, d'un latex, de particules métalliques colloïdales, de particules fluorescentes ou de particules colorées.

12. Dispositif selon la revendication 11, dans lequel la zone (iiia) comprend un tampon absorbant l'eau, contenant l'antigène recombinant génétique de tréponème de la syphilis, marqué d'un radio-isotope, d'un latex, de particules métalliques colloïdales, de particules fluorescentes ou de particules colorées.

13. Procédé d'analyse immunologique pour détecter un anticorps anti-tréponème de la syphilis, qui comprend les étapes consistant :
à appliquer une solution révélatrice contenant l'échantillon à analyser et un antigène recombinant génétique de tréponème de la syphilis, marqué d'un radioisotope, d'un latex, de particules métalliques colloïdales, de particules fluorescentes ou de particules colorées, à une zone (ia) du dispositif selon la revendication 10; et à détecter la présence ou l'absence d'anticorps anti-tréponème de la syphilis en détectant l'antigène marqué piégé dans la zone (iia), dont la quantité dépend de la quantité d'anticorps anti-tréponème de la syphilis à détecter.

14. Un procédé d'analyse immunologique pour détecter un anticorps anti-tréponème de la syphilis, qui comprend les étapes consistant :
à déposer ponctuellement avec une solution d'échantillon au niveau d'une zone (iiia) du dispositif selon la revendication 11 ou 12;
à fournir une solution révélatrice à la zone (ia) dudit dispositif; et
à détecter la présence ou l'absence d'anticorps anti-tréponème de la syphilis en détectant l'antigène marqué, piégé dans la zone (iia), dont la quantité dépend de la quantité d'anticorps anti-tréponème de la syphilis à détecter.
